# EUROPÄISCHE PATENTSCHRIFT

(11) **EP 1 870 100 B1**
(45) Veröffentlichungstag und Bekanntmachung des Hinweises auf die Patenterteilung: **11.01.2012**
(21) Anmeldenummer: 07115663.2
(22) Anmeldetag: 03.03.2003
(51) Int. Cl.: C07D 401/12

(54) **3-[(2-{[4-(Hexyloxycarbonylamino-imino-methyl)-phenylamino]methyl}-1-methyl-1H-benzimidazol-5-carbonyl)-pyridin-2-yl-amino]-propionsäure-ethylester Methansulfonat**
Ethyl 3-(2-(4-(hexyloxycarbonylamidino)phenylaminomethyl)-1-methyl-1H-benzimidazole-5-carbonyl)-2-pyridylamino)propionate methansulfonate
Méthanesulfonate d'éthyl-(2-(4-(héxyloxycarbonylamidino)phénylaminométhyl)-1-méthyl-1H-benzimidazole-5-carbonyl)-2-pyridylamino)propionate

(30) Priorität: 07.03.2002 DE 10209985; 30.09.2002 DE 10245624
(43) Veröffentlichungstag der Anmeldung: 26.12.2007
(62) Teilanmeldung aus: 03743368.7
(73) Patentinhaber: Boehringer Ingelheim Pharma GmbH & Co. KG, 55216 Ingelheim am Rhein (DE)
(72) Erfinder: Brauns, Ulrich, 88400, BIBERACH (DE); Hauel, Norbert, 88433, SCHEMMERHOFEN (DE)

(56) Entgegenhaltungen:
- WO-A-98/37075

## Beschreibung

Die Erfindung be schreibt eine oral zu applizierende Darreichungsform für den Wirkstoff 3-[(2-{[4-(Hexyloxycarbonylamino-imino-methyl)-phenylamino]-methyl}-1-methyl-1*H-*benzimidazol-5-carbonyl)-pyridin-2-yl-amino]-propionsäure-ethylester - Methansulfonat, Der Wirkstoff mit der chemischen Formel 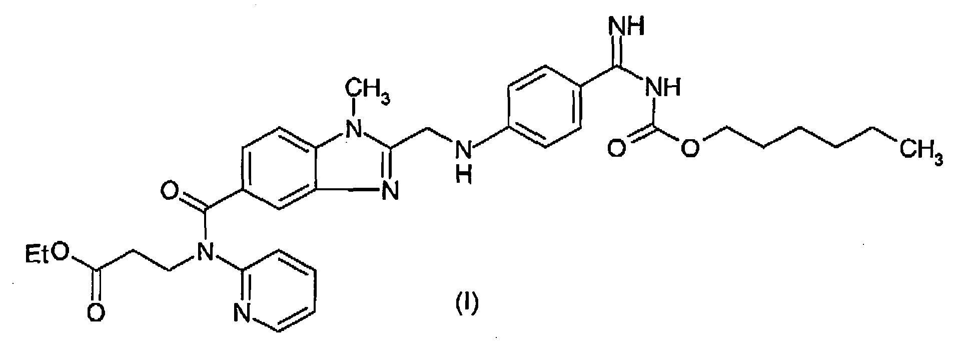 ist bereits aus der WO 98/37075, in der Verbindungen mit einer Thrombin-hemmenden und die Thrombinzeit verlängernden Wirkung offenbart werden, unter dem Namen 1-Methyl-2-[*N*-[4-(*N*-n-hexyloxycarbonylamidino)phenyl]-amino-methyl]-benzimidazol-5-yl-carbonsäure-*N*-(2-pyridyl)-*N*-(2-ethoxycarbonylethyl)-amid bekannt. Bei der Verbindung der Formel I handelt es sich um ein Doppel-Prodrug der Verbindung 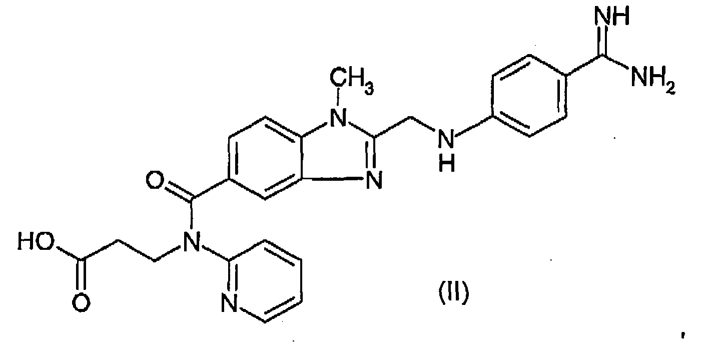 d.h. die Verbindung der Formel I wird erst im Körper in die eigentlich wirksame Verbindung, nämlich die Verbindung der Formel II, umgewandelt. Hauptindikationsgebiet der Verbindung der chemischen Formel I ist die postoperative Prophylaxe von tiefen Venenthrombosen.

Aufgabe der Erfindung ist es, eine verbesserte Formulierung zur oralen Anwendung für die Verbindung der Formel I (die im folgenden auch als "Wirkstoff" bezeichnet wird) bereitzustellen.

Überraschenderweise wurde nun gefunden, dass der Einsatz pharmazeutisch akzeptabler organischer Säuren mit einer Wasserlöslichkeit von > 1 g / 250 ml bei 20° C, vorzugsweise von > 1 g / 160 ml bei 25 °C, in festen oralen Darreichungsformen zu einer deutlich verbesserten Formulierung von 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester sowie von dessen pharmazeutisch verträglichen Methansulfonat-Salz führt.

Pharmazeutisch geeignete Säuren im Sinne dieser Erfindung sind beispielsweise Weinsäure, Fumarsäure, Bernsteinsäure, Zitronensäure, Äpfelsäure, Glutaminsäure und Asparaginsäure einschließlich deren Hydraten und sauren Salzen. Besonders geeignet im Sinne dieser Erfindung sind Weinsäure, Fumarsäure, Bernsteinsäure und Zitronensäure.

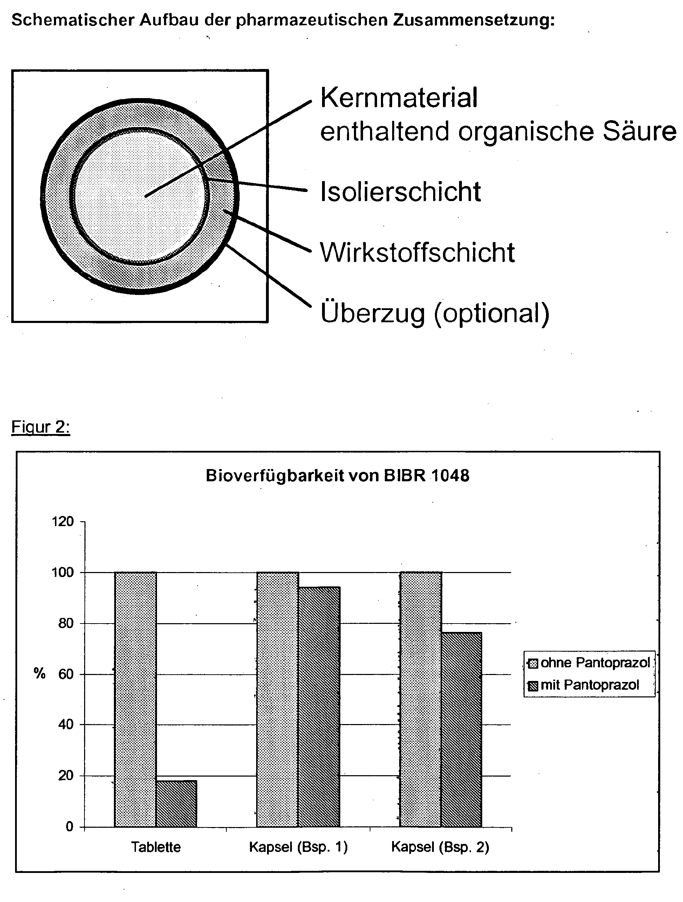

Eine bevorzugte form ist eine multipartikuläre Darreichungsform, in der die einzelnen Partikel wie in Figur 1 aufgebaut sind.

Figur 1 stellt den schematischen Aufbau der pharmazeutischen Zusammensetzung anhand eines Schnitts durch ein für die Herstellung der erfindungsgemäßen pharmazeutischen Zusammensetzung geeignetes Pellet dar. Der annähend kugelförmige/ sphärische Kernbereich dieses Pellets enthält/besteht aus der pharmazeutisch akzeptablen organischen Säure. Dann folgt eine Schicht, die den Säure-Kern von der wirkstoffhaltigen Schicht trennt, die sog. Isolierschicht. Die Isolierschicht wiederum ist von der ebenfalls kugelschalenförmigen Wirkstoffschicht umgeben, die ihrerseits von einem Überzug umschlossen sein kann, der die Abriebfestigkeit und die Lagerstabilität der Pellets erhöht.

Ein Vorteil der so aufgebauten Darreichungsform ist die räumliche Trennung von organischer Säure und Wirkstoff durch die Isolierschicht. Ein weiterer Vorteil des oben beschriebenen Aufbaus der Pellets ist die Tatsache, dass die organische Säure erst nach Applikation der Darreichungsform in Lösung geht und dann ein saures Mikroklima erzeugt, in dem sich der Wirkstoff auflösen kann.

Als Kernmaterial wird eine pharmazeutisch akzeptable organische Säuren mit einer Wasserlöslichkeit von > 1 g / 250 ml bei 20° C, wie z.B. Weinsäure, Fumarsäure, Bernsteinsäure, Zitronensäure, Äpfelsäure, Glutaminsäure und Asparaginsäure einschließlich deren Hydraten und sauren Salzen, verwendet, der gegebenenfalls ein geringer Anteil von 1 bis 10 Gew.-%, bevorzugt 3 bis 6 Gew.-% eines geeignete Bindemittels zugesetzt wird. Die Verwendung eines Bindemittels ist z.B. dann erforderlich, wenn die Säurestarter in einem Kessel-aufbau-verfahren hergestellt werden. Bei Verwendung eines Extrusions- oder Sphäronisierungs-Verfahrens benötigt man an Stelle von Bindemitteln andere technologische Hilfsstoffe, beispielsweise mikrokristalline Cellulose. Es ist auch denkbar, reine (100 %-ige) Säure als Startmaterial zu verwenden, wenn man diese in hinreichend enger Korngrößenverteilung beschaffen kann. Als pharmazeutisch akzeptable organische Säure werden bevorzugt Weinsäure, Fumarsäure, Bernsteinsäure oder Zitronensäure eingesetzt; besonders bevorzugt ist Weinsäure. Als Bindemittel können Gummi arabicum oder ein partial- oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder Kombinationen dieser Polymere verwendet werden; bevorzugt ist Gummi arabicum. Das sphärische Kernmaterial hat vorzugsweise einen mittleren Durchmesser von 0,4 - 1,5 mm. Der Gehalt der pharmazeutisch akzeptablen organischen Säure liegt üblicherweise zwischen 30 und 100 % im Kernmaterial, was einem Anteil von zwischen 20 und 90 %, vorzugsweise von zwischen 20 und 80 % im fertigen Pellet (d.h. in der pharmazeutischen Zusammensetzung) entspricht.

Zur Erhöhung der Lagerstabilität des Fertigproduktes ist es vorteilhaft, das Kernmaterial vor dem Auftrag des Wirkstoffes mit einer Isolierschicht basierend auf einem wasserlöslichen, pharmazeutisch akzeptablen Polymer zu beschichten. Als solches wasserlösliches Polymer kommen beispielsweise Gummi arabicum oder ein partial-oder vollsynthetischem Polymer aus der Gruppe der Hydroxypropylcellulosen, der Hydroxypropylmethylcellulosen, der Methylcellulosen, der Hydroxyethylcellulosen, der Carboxymethylcellulosen, der Polyvinylpyrrolidone, der Copolymerisate aus N-Vinylpyrrolidon und Vinylacetat, oder Kombinationen dieser Polymere in Frage. Bevorzugt wird Gummi arabicum oder eine Hydroxypropylmethylcellulose verwendet. Gegebenenfalls kann die Beschichtung mit dem wasserlöslichen, pharmazeutisch akzeptablen Polymer unter Zusatz geeigneter Weichmacher, Trennmittel und Pigmente erfolgen, wie beispielsweise Triethylcitrat, Tributylcitrat, Triacetin, Polyethylenglykole (Weichmacher), Talkum, Kieselsäure (Trennmittel), Titandioxid oder Eisenoxidpigmente (Pigmente).

Die Wirkstoffschicht enthält den Wirkstoff 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester(BIBR 1048) in Form seines pharmazeutisch akzeptablen Methansulfonat-Salzes sowie Binde- und gegebenenfalls Trennmittel. Als Bindemittel können beispielsweise Hydroxypropylcellulose, Hydroxypropylmethylcellulose, Methylcellulose, Hydroxyethylcellulose, Carboxymethylcellulose, Polyvinylpyrrolidon, Copolymerisate aus N-Vinylpyrrolidon und Vinylacetat oder Kombinationen dieser Polymere verwendet werden. Vorzugsweise wird Hydroxypropylcellulose oder Copolymerisate aus N-Vinylpyrrolidon und Vinylacetat eingesetzt. Der Zusatz von Trennmitteln wie z.B. Talkum oder Kieselsäure dient dazu, ein Zusammenwachsen der Partikel während des Prozesses zu verhindern. Der Wirkstoffgehalt beträgt 5 bis 60 %, vorzugsweise 10 bis 50 % der pharmazeutischen Zusammensetzung.

Die optionale äußerste Schicht, die zur Verminderung eines erhöhten Abriebs bei der Abfüllung in Kapseln und/oder zur Erhöhung der Lagerstabilität dient, besteht aus pharmazeutisch üblichen Filmbildnern, Weichmachern und gegebenenfalls Pigmenten. Als Filmbildner können beispielsweise Hydroxypropylcellulose, Hydroxypropylmethylcellulose, Methylcellulose, Polymerisate und Copolymerisate der Acryl- und Methacrylsäure und deren Estern, oder Kombinationen dieser Polymere verwendet werden. Als Weichmacher kommen unter anderem Triethylcitrat, Tributylcitrat, Triacetin oder Polyethylenglykole in Frage. Als Pigmente können z.B. Titandioxid oder Eisenoxidpigmente eingesetzt werden. Bevorzugt besteht der äußere Überzug aus Hydroxypropylmethylcellulose und / oder Methylcellulose, gegebenenfalls unter Zusatz von Polyethylenglykolen als Weichmacher.

Die Pellets können nach dem im folgenden beschriebenen Verfahren hergestellt werden:
Das säurehaltige Kernmaterial besteht entweder aus Kristallen der jeweils zur Verwendung kommenden organischen Säure oder vorteilhafter aus annähernd sphärischen Partikeln in der gewünschten Größe mit einem hohen Gehalt an organischer Säure, die mittels Verfahren hergestellt werden können, die in der pharmazeutischen Technologie bekannt und etabliert sind. In Frage kommen insbesondere der Aufbau des Kernmaterials in Kesselverfahren, auf Pelletiertellern, oder mittels Extrusion / Sphäronisierung. Anschließend kann das so erhaltenen Kernmaterial durch Sieben in Fraktionen mit dem gewünschten Durchmesser geteilt werden. Geeignetes Kernmaterial hat einen mittleren Durchmesser von 0,4 bis 1,5 mm, bevorzugt von 0,6 bis 0,8 mm.

Auf dieses säurehaltige Kernmaterial wird zunächst die Isolierschicht aufgetragen. Dies kann durch übliche Verfahren, z.B. durch Auftragen einer wäßrigen Dispersion des wasserlöslichen, pharmazeutisch akzeptablen Polymers gegebenenfalls unter Zusatz von Weichmachern, Trennmitteln und / oder Pigmenten in der Wirbelschicht, in Dragierkesseln oder in üblichen Filmcoatinganlagen, geschehen. Falls erforderlich, kann anschließend erneut gesiebt werden.

Daran anschließend wird der Wirkstoff aus einer bindemittel- und ggf. trennmittelhaltigen Dispersion aufgetragen. Das flüchtige Dispersionsmittel wird während des Prozesses und / oder daran anschließend durch Trocknen entfernt. Als Bindemittel in der Dispersion kann beispielsweise Hydroxypropylcellulose, Hydroxypropylmethylcellulose, Methylcellulose, Hydroxyethylcellulose, Carboxymethylcellulose, Polyvinylpyrrolidon, Copolymerisate aus N-Vinylpyrrolidon und Vinylacetat oder Kombinationen dieser Polymere verwendet werden. Vorzugsweise wird Hydroxypropylcellulose oder Copolymerisate aus N-Vinylpyrrolidon und Vinylacetat eingesetzt. Als Trennmittel eignen sich z.B. Talkum oder Kieselsäure; vorzugsweise wird Talkum verwendet. Als Dispersionsmittel kommen beispielsweise Ethanol, 2-Propanol, Aceton oder Mischungen dieser Lösungsmittel miteinander oder mit Wasser, bevorzugt 2-Propanol in Frage. Der Wirkstoffauftrag auf das Kernmaterial kann mittels in der pharmazeutischen Technologie bekannter und etablierter Verfahren z.B. in Dragierkesseln, konventionellen Filmcoatinganlagen oder in der Wirbelschicht erfolgen. Anschließend kann erneut ein Siebprozess durchgeführt werden.

Zur Verminderung eines erhöhten Abriebs bei der Abfüllung in Kapseln oder zur Erhöhung der Lagerstabilität kann das System abschließend noch mit einer Schicht aus pharmazeutisch üblichen Filmbildnern, Weichmachern und ggf. Pigmenten überzogen werden. Dies kann mit Hilfe von üblichen Verfahren erfolgen, wie sie bereits bei der Beschreibung des Aufbringens der Isolierschicht erwähnt wurden.

Bei Verwendung von Kernmaterial mit einem mittleren Durchmesser von 0,4 - 1,5 mm werden durch das oben beschriebene Verfahren wirkstoffhaltige Pellets erhalten, die anschließend beispielsweise in Hartkapseln abgefüllt werden können. Dazu wird eine der Dosierung entsprechende Vielzahl dieser Einheiten auf Standard-Kapselfüllmaschinen in Hartkapseln gefüllt. Als geeignete Hartkapseln kommen beispielsweise Hartgelatinekapseln oder Hartkapseln aus Hydroxypropylmethylcellulose (HPMC) in Frage; bevorzugt sind HPMC-Kapseln. Der Wirkstoffgehalt der pharmazeutischen Zusammensetzung beträgt 5 bis 60 %, vorzugsweise 10 bis 50 %; der Gehalt der pharmazeutisch akzeptablen organischen Säure liegt üblicherweise zwischen 20 und 90 %, vorzugsweise zwischen 20 und 80 %.

Soweit nicht anders vermerkt, handelt es sich bei den Prozentangaben stets um Gewichtsprozente. Sämtliche Angaben über den Wirkstoffgehalt beziehen sich, sofern nicht anders angegeben, auf die Wirkstoffbase der Formel I (nicht auf ein bestimmtes Salz).

### Klinische Prüfungen

Bei den ersten Probanden-Versuchen mit konventionellen Tabletten enthaltend die Verbindung der Formel I war festgestellt worden, daß hochvariable Plasmaspiegel bis hin zu vereinzelten Malabsorptionen auftraten. Die Variabilität der Plasmaspiegelverläufe ist nach Applikation der Verbindung der Formel als oral applizierte Lösung deutlich niedriger; eine Malabsorption wurde hierbei nicht beobachtet.

Untersuchungen haben gezeigt, dass die Verbindung der Formel I in Wasser bei niedrigen pH-Werten verhältnismäßig gut löslich ist, während sie bei pH-Werten über 5 gemäß der Definition des europäischen Arzneibuches praktisch unlöslich ist. Daher wurde den Probanden in einem Behandlungsast der klinischen Prüfungen Pantoprazol verabreicht, welches dazu dient, einen erhöhten Magen-pH hervorzurufen.

Beispielsweise wurden die pharmazeutischen Zusammensetzungen gemäß der Beispiele 1 und 2 auf ihre Bioverfügbarkeit im Vergleich zu einer konventionellen Tablette hin getestet.

Dazu wurde die gemäß Beispiel 1 hergestellte Formulierung mit einem Gehalt an Wirkstoffbase von 50 mg pro Kapsel an insgesamt 15 Probanden im Hinblick auf ihre Bioverfügbarkeit klinisch geprüft. In einem Behandlungsast erhielten die Probanden die Zusammensetzung nüchtern per os (= orale Gabe) ohne eine Vorbehandlung. In einem weiteren Behandlungsast wurden die gleichen Probanden vor per os - Applikation der Zusammensetzung zur Erhöhung des Magen-pH drei Tage lang mit 40 mg Pantoprazol b.i.d. (= zwei mal täglich) per os vorbehandelt; die Behandlung mit Pantoprazol wurde während der Verabreichung der erfindungsgemäßen Formulierung fortgeführt.

Das Ausmaß der Absorption wurde über die quantitative Bestimmung der Urinausscheidung des wirksamen Metaboliten der Formel II ermittelt.

Die relative Bioverfügbarkeit nach Vorbehandlung mit Pantoprazol lag im Vergleich zur Applikation ohne vorhergehende Vorbehandlung im Mittel bei 94 %.

Unter vergleichbaren Applikationsbedingungen liegt die relative Bioverfügbarkeit (basierend auf der Fläche unter der Plasmakonzentrations-Zeit Kurve) einer 50 mg Wirkstoff enthaltenden Tablette, die nach dem Stand der Technik entwickelt und hergestellt wurde und keine wasserlösliche organische Säure enthält, nach entsprechender Vorbehandlung mit Pantoprazol bei 18 %. Die folgende Liste gibt die genaue Zusammensetzung der verwendeten Tablette an:

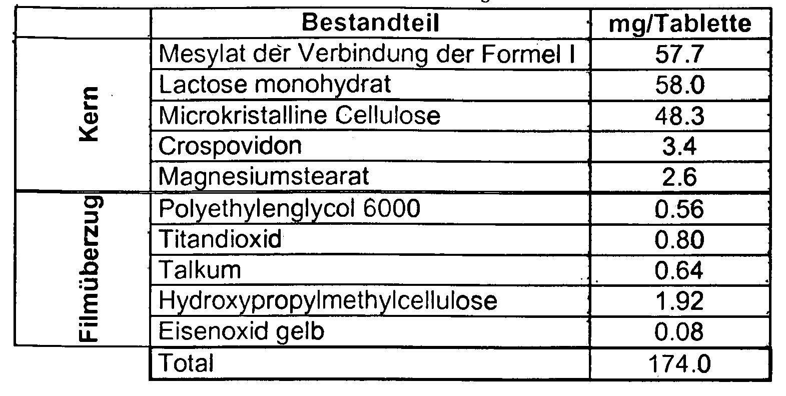

Die relative Bioverfügbarkeit wurde durch Verwendung der erfindungsgemäßen Formulierung also ca. um den Faktor 5 verbessert.

Die gemäß Beispiel 2 hergestellte Formulierung mit einem Gehalt an Wirkstoffbase von 50 mg pro Kapsel wurde ebenfalls an insgesamt 15 Probanden im Hinblick auf ihre Bioverfügbarkeit klinisch geprüft. In einem Behandlungsast erhielten die Probanden die Zusammensetzung nüchtern per os ohne eine Vorbehandlung. In einem weiteren Behandlungsast wurden die gleichen Probanden vor per os - Applikation der Zusammensetzung zur Erhöhung des Magen-pH drei Tage lang mit 40 mg Pantoprazol b.i.d. per os vorbehandelt; die Behandlung mit Pantoprazol wurde während der Verabreichung der erfindungsgemäßen Formulierung fortgeführt.

Das Ausmaß der Absorption wurde über die quantitative Bestimmung der Urinausscheidung des wirksamen Metaboliten der Formel II ermittelt.

Die relative Bioverfügbarkeit nach Vorbehandlung mit Pantoprazol lag im Vergleich zur Applikation ohne vorhergehende Vorbehandlung im Mittel bei 76 %.

Unter vergleichbaren Applikationsbedingungen liegt die relative Bioverfügbarkeit (basierend auf der Fläche unter der Plasmakonzentrations-Zeit Kurve) einer 50 mg Wirkstoff enthaltenden Tablette, die nach dem Stand der Technik entwickelt und hergestellt wurde und keine wasserlösliche organische Säure enthält, nach entsprechender Vorbehandlung mit Pantoprazol bei 18 %. Die folgende Liste gibt die genaue Zusammensetzung der verwendeten Tablette an:

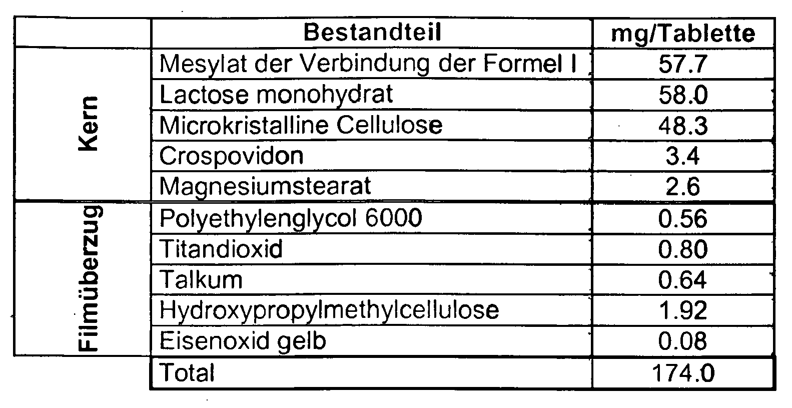

Durch die Verwendung der erfindungsgemäßen Formulierung wurde die relative Bioverfügbarkeit des Wirkstoffs gegenüber herkömmlicher Formulierungen also ca. um den Faktor 4 verbessert. Die Bioverfügbarkeit der beiden erfindungsgemäßen Formulierungen im Vergleich zur oben beschriebenen Tablette mit bzw. ohne gleichzeitige Gabe von Pantoproazol ist in Figur 2 graphisch dargestellt.

Die klinischen Prüfung zeigen einen weiteren Vorteil des erfindungsgemäßen Darreichungsform enthaltend die Verbindung der Formel I, der darin besteht, eine ausreichende, gegenüber einer konventionellen pharmazeutischen Zubereitung verbesserte sowie vom Magen-pH weitgehend unabhängige Bioverfügbarkeit des Wirkstoffs zu gewährleisten, Schwankungen der Bioverfügbarkeit des Wirkstoffs zu vermindern und Malabsorptionen zu verhindern. Eine weitere vorteilhafte Eigenschaft der erfindungsgemäßen pharmazeutischen Zusammensetzung ist ihre Eignung für alle Patienten, also auch für solche Personen, bei denen der Magen-pH durch normale physiologische Variabilität, durch eine Krankheit oder durch eine Komedikation mit Arzneimitteln, die den Magen-pH erhöhen, erhöht ist.

Die Dosierung beträgt bei oraler Gabe zweckmäßigerweise 25 bis 300 mg der Wirkstoffbase (pro Kapsel), vorzugsweise 50 bis 200 mg, besonders bevorzugt 75 bis 150 mg der Wirkstoffbase, jeweils 1 bis 2 mal täglich.

Das bevorzugte Säure-Wirkstoffverhältnis beträgt ca. 0,9 : 1 bis ca. 4 : 1, besonders bevorzugt ist ein Verhältnis von ca. 1:1 und von ca. 3:1. Bevorzugt wird mindestens 1 Equivalent Säure pro Mol der Verbindung der Formel I verwendet. Die Obergrenze von ca. 4:1 (Säure zu Wirkstoff), wird in der Regel durch die maximal akzeptable Größe der Darreichungsform bei den gewünschten Dosierungen bestimmt (Menge der Pellets pro Kapsel).

Die nachfolgenden Beispiele sollen die Erfindung näher erläutern:

### Beispiel 1

| | Prozentuale Zusammensetzung | | | | pro Kapseln [mg] | pro Kapsel [mg] |
|---|---|---|---|---|---|---|
| | Kernmaterial | Isoliefschicht | Wirkstoffschicht | Gesamt | | |
| Weinsäure | 61,3 | - | - | 61,3 | 176,7 | 353,4 |
| Gummi arabicum | 3,1 | 2,8 | | 5,9 | 17,0 | 34,0 |
| Talkum | - 5,6 | | 3,2 | 8,8 | 25,4 | 50,7 |
| Hydroxypropylcellulose | - | - | 4,0 | 4,0 | 11,5 | 23,1 |
| Wirkstoff (Mesylat der Verbindung der Formel I) | - | - | 20,0 | 20,0 | 57,7* | 115,3** |
| Summe | | | | 100,0 | 288,3 | 576,5 |

| | | | | | | |
|---|---|---|---|---|---|---|
| *) entspricht 50 mg der Verbindung der Formel 1 (Wirkstoffbase) **) entspricht 100 mg der Verbindung der Formel 1 (Wirkstoffbase) | | | | | | |

### a) Aufbau von weinsäurehaltigem Kernmaterial

| Zusammensetzung: | |
|---|---|
| Gummi arabicum | 1 Gewichtsteil |
| Weinsäure | 20 Gewichtsteile |

In 4 Gewichtsteilen gereinigtem Wasser von 50°C wird unter Rühren 1 Gewichtsteil Gummi arabicum gelöst. In dieser Lösung werden anschließend unter Rühren 5 Gewichtsteile Weinsäure gelöst.

In einer geeigneten, mit einer Zu- und Ablufteinrichtung versehenen Dragieranlage werden 8,3 Gewichtsteile Weinsäurekristalle mit einer mittleren Partikelgröße von 0,4 bis 0,6 mm vorgelegt und der Kessel in Rotation versetzt. Bei einer Zulufttemperatur von 60° - 80° C werden die Weinsäurekristalle im Intervallbetrieb mit der Weinsäure-Gummi arabicum - Lösung besprüht und mit insgesamt 6,7 Gewichtsteilen pulverförmiger Weinsäure bepudert, so dass annähernd sphärische Partikel entstehen.

Das sphärische Weinsäurekernmaterial wird anschließend im rotierenden Kessel bei einer Zulufttemperatur von 60° - 80° C nachgetrocknet.

Das Kernmaterial wird über eine Taumelsiebmaschine mit Siebböden mit einer nominalen Maschenweite von 0,6 und 0,8 mm fraktioniert. Die Gutfraktion zwischen 0,6 und 0,8 mm wird im weiteren Prozess verwendet.

### b) Isolierung des weinsäurehaltigen Kernmaterials

| Zusammensetzung: | |
|---|---|
| Weinsäurehaltiges Kernmaterial | 23 Gewichtsteile |
| Gummi arabicum | 1 Gewichtsteil |
| Talkum | 2 Gewichtsteile |

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile weinsäurehaltiges Kernmaterial bei einer Zulufttemperatur von 35° - 40° C mit der Gummi arabicum-Talkum - Dispersion im Unterbettsprühverfahren besprüht.

Das isolierte weinsäurehaltige Kernmaterial wird anschließend im Umlufttrockenschrank bei 40° C über 8 Stunden nachgetrocknet.

Zur Entfernung von Agglomeraten wird das getrocknete isolierte weinsäurehaltige Kernmaterial mit einem Sieb mit der nominalen Maschenweite 1,0 mm gesiebt. Die Gutfraktion (Korngröße < 1 mm) wird weiterverarbeitet.

### c) Aufbau der Wirkstoffschicht

| Zusammensetzung: | |
|---|---|
| Isoliertes weinsäurehaltiges Kernmaterial | 91 Gewichtsteile |
| Hydroxypropylcellulose | 5 Gewichtsteile |
| Talkum | 4 Gewichtsteile |
| Wirkstoff (Mesylat von BIBR 1048) | 25 Gewichtsteile |

In 168 Gewichtsteilen 2-Propanol wird Hydroxypropylcellulose unter Rühren gelöst und anschließend in dieser Lösung der Wirkstoff und Talkum unter Rühren dispergiert.

In einer Wirbelschichtprozessanlage werden 91 Gewichtanteile isoliertes weinsäurehaltiges Kernmaterial bei einer Zulufttemperatur von 20° - 30° C mit der wirkstoffhaltigen Dispersion im Unterbettsprühverfahren besprüht.

Die wirkstoffhaltigen Pellets werden anschließend im Umlufttrockenschrank bei 35° C über 8 Stunden nachgetrocknet.

Zur Entfernung von Agglomeraten werden die wirkstoffhaltigen Pellets mit einem Sieb mit der nominalen Maschenweite 1,25 mm gesiebt. Die Gutfraktion (Korngröße < 1,25 mm) wird weiterverarbeitet.

### d) Abfüllung in Kapseln

Eine jeweis 50 bzw. 100 mg Wirkstoffbase enthaltende Menge an wirkstoffhaltigen Pellets wird mittels einer Kapselfüllmaschine in Hartgelatinekapseln oder HPMC-Kapseln der Größe 1 bzw. der Größe 0 elongated abgefüllt.

### Beispiel 2

| | Prozentuale Zusammensetzung | | | | pro Kapsel [mg] | pro Kapsel [mg] |
|---|---|---|---|---|---|---|
| | kernmaterial | Isolierschicht | Wirkstoffschicht | Gesamt | | |
| Weinsäure | 38,5 | - | - | 38,5 | 55,5 | 166,5 |
| Gummi arabicum | 1,9 | 1,7 | | 3,6 | 5,2 | 15,6 |
| Talkum | - 3,5 | | 6,4 | 9,9 | 14,3 | 42,8 |
| Hydroxypropylcellulose | - | - | 8,0 | 8,0 | 11,5 | 34,6 |
| Wirkstoff (Mesylat der Verbindung der Formel I) | - | - | 40,0 | 40,0 | 57,7* | 173,0** |
| Summe | | | | 110,0 | 144,2 | 432,5 |

| | | | | | | |
|---|---|---|---|---|---|---|
| *) entspricht 50 mg der Verbindung der Formel 1 (Wirkstoffbase) **) entspricht 150 mg der Verbindung der Formel 1 (Wirkstoffbase) | | | | | | |

### a) Aufbau von weinsäurehaltigem Kernmaterial

| Zusammensetzung: | |
|---|---|
| Gummi arabicum | 1 Gewichtsteil |
| Weinsäure | 20 Gewichtsteile |

In 4 Gewichtsteilen gereinigtem Wasser von 50°C wird unter Rühren 1 Gewichtsteil Gummi arabicum gelöst. In dieser Lösung werden anschließend unter Rühren 5 Gewichtsteile Weinsäure gelöst.

In einer geeigneten, mit einer Zu- und Ablufteinrichtung versehenen Dragieranlage werden 8,3 Gewichtsteile Weinsäurekristalle mit einer mittleren Partikelgröße von 0,4 bis 0,6 mm vorgelegt und der Kessel in Rotation versetzt. Bei einer Zulufttemperatur von 60° - 80° C werden die Weinsäurekristalle im Intervallbetrieb mit der Weinsäure-Gummi arabicum - Lösung besprüht und mit insgesamt 6,7 Gewichtsteilen pulverförmiger Weinsäure bepudert, so dass annähernd sphärische Partikel entstehen.

Das sphärische Weinsäurekernmaterial wird anschließend im rotierenden Kessel bei einer Zulufttemperatur von 60° - 80° C nachgetrocknet.

Das Kernmaterial wird über eine Taumelsiebmaschine mit Siebböden mit einer nominalen Maschenweite von 0,6 und 0,8 mm fraktioniert. Die Gutfraktion zwischen 0,6 und 0,8 mm wird im weiteren Prozess verwendet.

### b) Isolierung des weinsäurehaltigen Kernmaterials

| Zusammensetzung: | |
|---|---|
| Weinsäurehaltiges Kernmaterial | 23 Gewichtsteile |
| Gummi arabicum | 1 Gewichtsteil |
| Talkum | 2 Gewichtsteile |

In einer Mischung aus 6,7 Gewichtsteilen Ethanol 96 % und 13,5 Gewichtsteilen gereinigtem Wasser wird unter Rühren 1 Gewichtsanteil Gummi arabicum gelöst. Anschließend werden in der Lösung unter Rühren 2 Gewichtsanteile Talkum dispergiert.

In einer Wirbelschichtprozessanlage werden 23 Gewichtanteile weinsäurehaltiges Kernmaterial bei einer Zulufttemperatur von 35° - 40° C mit der Gummi arabicum-Talkum - Dispersion im Unterbettsprühverfahren besprüht.

Das isolierte weinsäurehaltige Kernmaterial wird anschließend im Umlufttrockenschrank bei 40° C über 8 Stunden nachgetrocknet.

Zur Entfernung von Agglomeraten wird das getrocknete isolierte weinsäurehaltige Kernmaterial mit einem Sieb mit der nominalen Maschenweite 1,0 mm gesiebt. Die Gutfraktion (Korngröße < 1 mm) wird weiterverarbeitet.

### c) Aufbau der Wirkstoffschicht

| Zusammensetzung: | |
|---|---|
| Isoliertes weinsäurehaltiges Kernmaterial | 57 Gewichtsteile |
| Hydroxypropylcellulose | 10 Gewichtsteile |
| Talkum | 8 Gewichtsteile |
| Wirkstoff (Mesylat von BIBR 1048) | 50 Gewichtsteile |

In 335 Gewichtsteilen 2-Propanol wird Hydroxypropylcellulose unter Rühren gelöst und anschließend in dieser Lösung der Wirkstoff und Talkum unter Rühren dispergiert.

In einer Wirbelschichtprozessanlage werden 91 Gewichtanteile isoliertes weinsäurehaltiges Kernmaterial bei einer Zulufttemperatur von 20° - 30° C mit der wirkstoffhaltigen Dispersion im Unterbettsprühverfahren besprüht.

Die wirkstoffhaltigen Pellets werden anschließend im Umlufttrockenschrank bei 35° C über 8 Stunden nachgetrocknet.

Zur Entfernung von Agglomeraten werden die wirkstoffhaltigen Pellets mit einem Sieb mit der nominalen Maschenweite 1,25 mm gesiebt. Die Gutfraktion (Korngröße < 1,25 mm) wird weiterverarbeitet.

### d) Abfüllung in Kapseln

Eine jeweils 50 bzw. 150 mg Wirkstoffbase enthaltende Menge an wirkstoffhaltigen Pellets wird mittels einer Kapselfüllmaschine in Hartgelatinekapseln oder HPMC-Kapseln der Größe 2 bzw. der Größe 0 abgefüllt.

### Beispiel 3

### Herstellung von 3-[(2-{[4-(Hexyloxycarbonylamino-imino-methyl)-phenylamino]-methyl}-1-methyl-1H-benzimidazol-5-carbonyl)-pyridin-2-yl-amino]-propionsäure-ethylester- Methansulfonat

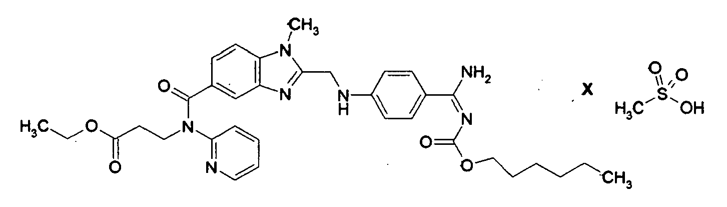

Zu einer Lösung von 3139 mg (5.0 mMol) 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester - Base (wie in der WO 98/37075 beschrieben hergestellt), in 250 ml Essigsäureethylester wurde eine Lösung von 5.0 mMol Methansulfonsäure in 25 ml Essigsäureethylester unter Rühren bei Raumtemperatur tropfenweise zugegeben. Nach wenigen Minuten begann das Produkt auszukristallisieren. Es wurde noch eine Stunde lang bei Raumtemperatur und eine weitere unter Eiskühlung gerührt, dann der Niederschlag abgesaugt, mit ca. 50 ml Essigester und 50 ml Diethylether gewaschen und bei 50°C im Umluft-Trockenschrank getrocknet.
Ausbeute: 94% der Theorie
Schmelzpunkt: 178 - 179°C
C₃₄H₄₁N₇O₅ x CH₄SO₃ (723.86)

| | | | | | | | | |
|---|---|---|---|---|---|---|---|---|
| Elementar-Analyse: ber.: | C | 58.07% | H | 6.27% | N | 13.55% | S | 4.43% |
| gef:: | | 58.11% | | 6.30% | | 13.50% | | 4.48% |

## Patentansprüche

1. 3-[(2-{[4-(Hexyloxycarbonylamino-imino-methyl)-phenylamino]-methyl}-1-methyl-1*H*-benzimidazol-5-carbonyl)-pyridin-2-yl-amino]-propionsäure-ethylester-. Methansulfonat.

## Claims

1. Ethyl 3-[(2-{[4-(hexyloxycarbonylamino-imino-methyl)-phenylamino]-methyl}-1-methyl-1H-benzimidazole-5-carbonyl)-pyridin-2-yl-amino]-propionate methanesulphonate.

## Revendications

1. Méthanesulfonate d'éthylester d'acide 3-[(2-{[4-(hexyloxy-carbonylamino-imino-méthyl)-phénylamino]-méthyl}-1-méthyl-1H-benzimidazol-5-carbonyl)-pyridin-2-yl-amino]-propionique.
